(19) Europäisches Patentamt
European Patent Office
Office européen des brevets

(11) Publication number: 0 034 012
B1

(12) **EUROPEAN PATENT SPECIFICATION**

(45) Date of publication of patent specification: **12.09.84**

(51) Int. Cl.³: **C 07 C 119/06,**
C 07 C 131/00, A 01 N 35/10

(21) Application number: **81300350.6**

(22) Date of filing: **27.01.81**

(54) **2-nitro-5-(substituted-phenoxy) phenylalkanone oxime and imine derivatives as herbicides.**

(30) Priority: **01.02.80 US 117731**

(43) Date of publication of application:
**19.08.81 Bulletin 81/33**

(45) Publication of the grant of the patent:
**12.09.84 Bulletin 84/37**

(84) Designated Contracting States:
**AT BE CH DE FR IT LI NL SE**

(56) References cited:
**BE-A- 870 068**
**GB-A-2 049 695**

(73) Proprietor: **RHONE-POULENC AGROCHIMIE**
**14-20, rue Pierre Baizet**
**F-69009 Lyon (FR)**

(72) Inventor: **Theissen, Robert James**
**474 Van Holten Road**
**Bridgewater New Jersey 08807 (US)**

(74) Representative: **Brachotte, Charles et al**
**RHONE-POULENC AGROCHIMIE 14-20, Rue**
**Pierre Baizet B.P. 9163**
**F-69263 Lyon Cedex 1 (FR)**

**EP 0 034 012 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with herbicidal 2-nitro-5-(substituted-phenoxy) phenylalkanone oxime and imine derivatives.

U.S. Patent Nos. 3,652,645; 3,784,635; 3,873,302; 3,983,168 and 3,907,866 describe certain phenoxy benzoic acid derivatives which are useful as herbicides. We have now discovered further phenoxy benzoic acid derivatives which have good activity as herbicides.

This invention relates to certain herbicidal compounds falling within the general formula:

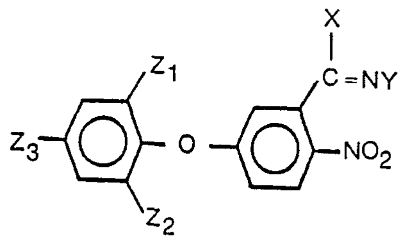

wherein

$Z_1$ is halogen,

$Z_2$ is selected from halogen and hydrogen, and

$Z_3$ is a polyhalo$_{1-9}$ alkyl$_{1-4}$ group such as $CF_3$, $CHF_2$, $C_4F_9$, $CF_2CH_2CH_3$ and $CH_2Cl$.

X is selected from the group consisting of hydrogen, cyano, alkoxy of 1 to 6 carbons, alkyl of 1 to 6 carbons.

Y is selected from the group consisting of hydroxy, alkali metal oxide (e.g., —ONa, —OK), N-alkylaminocarboxy and N,N-dialkylaminocarboxy where each alkyl group is of 1 to 6 carbons, 1-(alkoxycarbonyl) alkoxy where each alkoxy is of 1 to 6 carbons, alkyl of 1 to 6 carbons, epoxyalkoxy where the alkoxy is of 3 to 6 carbons.

The alkyl and alkoxy groups referred to herein may be of branched or straight carbon chains.

In accordance with the foregoing formula, preferable substituents include $Z_1$ is chloro, $Z_3$ is $CF_3$ and $Z_2$ is hydrogen.

The present nitrogen-containing compounds are prepared from the corresponding 2-nitro-5-(substituted-phenoxy) phenyl alkanone derivatives. One method for preparing the alkanones is the Ullman ether synthesis between the alkali metal (e.g., Na, K) salt of a suitable substituted phenol, e.g., m-hydroxy benzaldehyde or m-cresol with an active halogen-substituted aromatic, e.g., 3,4-dichlorobenzotrifluoride. The intermediate obtained may be nitrated and subsequently derivatized by known procedures. Where m-cresol is used as a starting material, the product obtained can be oxidized and subsequently nitrated before the aforementioned derivatization. The alkanone of the formula

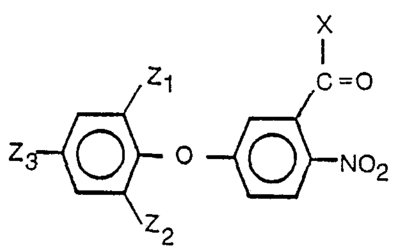

is reacted with a nitrogenous compound $H_2NY$ to form the final product.

The following Examples are given by way of illustration.

*Compound 1*

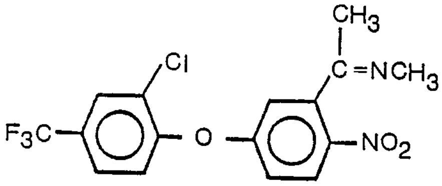

Preparation of 5-[2-chloro-4-(trifluoromethyl)phenoxy]-2-nitrophenyl ethanone methylimine.

To a stirred solution of 5-[2-chloro-4-(trifluoromethyl) phenoxy]-2-nitrophenyl ethanone (3.3 g.,

0.0092 mole) in absolute ethanol (30 ml) was bubbled methylamine for 0.5 hour as the temperature rose to about 40°C. The solution was stirred for 12 hours at room temperature and then heated to reflux for 4 hours. The solvent was stripped to give 3.0 of a brown oil. Recrystallization from methanol/water gave a brown solid (1.2 g., mp. 133—7°C).

I.R. (nujol): C=O absent

*Compound 2*

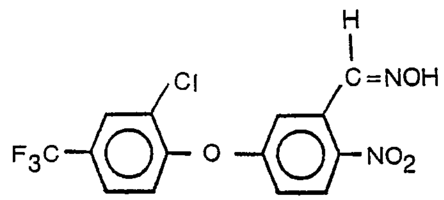

Preparation of 5-[2-chloro-4-(trifluoromethyl) phenoxy]-2-nitrobenzaldehyde oxime.

To a solution of hydroxylamine hydrochloride (0.33 g., 0.0048 mole) and sodium acetate (0.394 g., 0.0048 mole) in 2—3 ml of water was added a hot solution of 5-[2-chloro-4-(trifluoromethyl) phenoxy]-2-nitrobenzaldehyde (1.5 g., 0.00434 mole) in ethanol (10—12 ml). The clear yellow solution was heated for 1.5 hours and then the solvent was stripped on a rotary evaporator to give a yellow waxy solid which solidified giving 1.5 g., m.p. 111—115°C. Recrystallization from ethanol/water gave a solid mp. 114—118°C.

| | |
|---|---|
| I.R. (KBr): | C=O absent |
| NMR ($CDCl_3$): | Singlet 8.85 ppm (1H) |
| | Doublet 8.30 ppm (J=4.5 Hz, 1H) |
| | Complex multiplet 7.0—8.0 ppm (7H) |

*Compound 3*

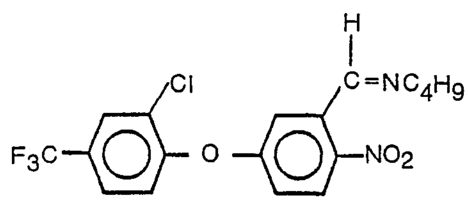

5-[2-chloro-4-(trifluoromethyl) phenoxy]-2-nitrobenzaldehyde t-butylimine was prepared in a manner similar to that shown for Compound 1. The product was an oily semi-solid.

| | |
|---|---|
| I.R. (neat): | C=O absent |
| NMR ($CDCl_3$): | Singlet 1.34 ppm (9H) |
| | Complex muliplet 6.9—8.4 ppm (6H) |
| | Singlet 8.9 ppm (1H) |

Other illustrative compounds of the present invention are shown below in accordance with the following formula:

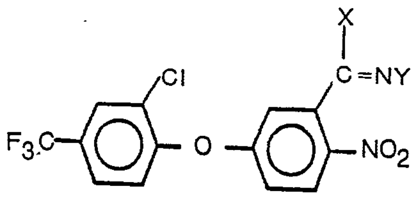

| X | Y |
|---|---|
| H | $-ONa$ |
| H | 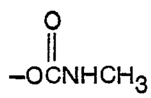 |
| H | 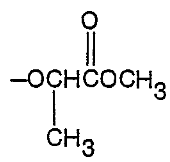 |
| $-CN$ | 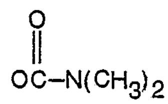 |
| $-OC_2H_5$ | 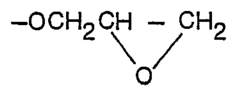 |

The compounds of this invention can be applied in various ways to achieve herbicidal action. They can be applied per se, as solids or in vaporized form, but are preferably applied as the toxic components in pesticidal compositions of the compound and a carrier. These compositions are preferably applied directly to the soil and often incorporated therewith. The compositions can be applied as granulars or dusts; as liquid sprays, or as gas-propelled sprays and can contain, in addition to a carrier, additives such as emulsifying agents, binding agents, gases compressed to the liquid state, odorants, stabilizers, and the like. A wide variety of liquid and solid carriers can be used. Non-limiting examples of solid carriers include talc, bentonite, diatomaceous earth, pyrophyllite, fullers earth, gypsum, flours derived from cotton seeds and nut shells, and various natural and synthetic clays having a pH not exceeding about 9.5. Non-limiting examples of liquid carriers include water, organic solvents such as alcohols, ketones, light oils, and medium oils and vegetable oils such as cottonseed oil.

In practice, herbicidal application is measured in terms of pounds of herbicide applied per acre. The compounds of this invention are effective herbicides when applied in herbicidal amounts, e.g. at rates between 0.2 and 10 kg. per hectare.

The pesticidal compositions comprising the active ingredient and the carrier may be supplied either as ready-to-use compositions or as concentrates. Concentrates generally contain a higher proportion of the active ingredient than the ready-to-use compositions and accordingly, require dilution prior to use. Both the ready-to-use compositions and the concentrates may be in liquid or solid form, i.e. with the active ingredient blended with a liquid or solid carrier, respectively.

The amount of active ingredient in the composition will depend, primarily, upon whether the composition is a concentrate or a ready-to-use composition. Concentrates will generally contain from 5 to 95% by weight, preferably 10 to 80% by weight, of the active ingredient. The concentration of active ingredient in the ready-to-use compositions will vary according to the method of application for the composition in question and to the desired application rate. In general, ready-to-use compositions will contain from 0.001 to 1, preferably 0.01 to 0.1 percent by weight of the active ingredient. Thus, the compositions may contain from 0.001 to 95% by weight of active ingredient, the actual amount depending upon the nature of the composition and the method by which it shall be applied.

Concentrates may be either liquid or solid and are usually extendable with water to form emulsions or suspensions containing a smaller proportion of the active ingredient. Alternatively, liquid or solid concentrates may be extended with liquid or solid carriers to give the final ready-to-use composition. Liquid concentrates preferably comprise the active ingredient and an emulsifying agent

dissolved in a liquid solvent, e.g. an organic solvent of the type mentioned above. The emulsifier is suitably an anionic, cationic or non-ionic emulsifier, e.g. sodium dodecylbenzenesulfonate or an ethylene oxide derivative of an alkyl phenol, a mercaptan, an amine or a carboxylic acid. Liquid concentrates may advantageously contain from 10 to 30 weight percent, e.g. 25 weight percent of the active ingredient, e.g. 1 kg. of active ingredient per 4 kg. of concentrate.

Wettable powders are another preferred form of concentrate and these suitably comprise the active ingredient, a finely-divided solid carrier and at least one surfactant to impart wettability or dispersability. Solid carriers which are suitable for preparing wettable powder formulations may be either organic or inorganic in nature. Suitable organic carriers are soybean, walnut, or wood flour or tobacco dust, and suitable inorganic ones are clays of the bentonite, kaolinite, or fuller's earth types; silicas such as diatomaceous earth; silicates such as talc, pyrophyllite, alkaline earth silicates; and calcium and magnesium carbonates. The carrier may be a single substance or a mixture of finely-divided solids. A surfactant or mixture of surfactants is generally present in an amount of 1 to 10 percent by weight of the wettable powder formulation. Suitable dispersing agents are sodium formaldehydenaphthalene sulfonate or sodium lignin sulfonate. Wetting agents include higher alkylaryl sulfonates ("higher alkyl" meaning alkyl of at least 8 carbon atoms) such as calcium dodecylbenzenesulfonate, alcohol sulfates, alkyl-phenoxyethyoxyethoxyethyl sodium sulfonates, sodium dioctyl sulfosuccinate, and ethylene oxide adducts with fatty alcohols, fatty acids, or with higher alkyl-phenols, such as octylphenoxypolyethoxyethanol. Sticking or spreading agents may be included such as glycerol mannitan laurate or a condensate of polyglycerol and oleic acid modified with phthalic anhydride. The active ingredient content of the wettable powder may be in the range of 20 to 80% by weight; however, the preferred range of concentrations is 50% to 80%.

Dusts may be made by incorporating the active ingredient into a solid carrier, such as finely powdered clays, talc, silica, and synthetic silicates, alkaline earth carbonates, and diluents of natural origin such as tobacco dust or walnut shell flour. Granular formulations can be made from similar type solid carriers except that the particle size is larger, in the range of 15 to 60 mesh (U.S. Standard Sieve Series). A small amount of dispersing agent may be incorporated in these solid formulations. The concentration of active ingredients in these dust or granular formulations may be in the range of 1 to 20% by weight. The solid carriers used in these formulations may be essentially inert or they may consist wholly or in part of fertilizing materials such as ammonium sulfate, or other ammonium salts, urea, calcium phosphates, potassium chloride or dried blood.

One particularly convenient method for making solid formulations is to treat the solid carrier with the active ingredients dissolved in a solvent and allow the solvent to evaporate off. This results in the carrier which is usually in the form of finely-divided particles, being impregnated with the active ingredients. Another method is to apply the mixture of active ingredients in the molten state or by spraying.

The concentration of the active ingredient in the final ready-to-use composition will depend not only upon the application rate desired (generally in the range of 0.2 to 10 kg. per hectare, as mentioned above), but also upon the application method which is to be used. Wettable powders and liquid concentrations are usually applied as aqueous sprays and applied at application rates varying from about 10 to 1500 liters per hectare. With ground equipment, the application rate will generally be in the range of 100 to 500 liters per hectare, whereas aerial spray equipment will generally apply 15 to 80 liters per hectare.

## Herbicidal Effectiveness

*Method of Propagating Test Species*

Crop and weed species are planted in 8″ × 10″ disposable fiber flats containing potting soil to provide each flat with a 4″ row of all test species. Crop species consist of field corn (CN), cotton (CT), and soybeans (SB). The weed species consist of foxtail millet (FM), green foxtail (GF), crabgrass (CG), velvetleaf (VL), cocklebur (CB), wild mustard (WM) and pigweed (PW).

Cotton, corn, soybean, and cocklebur plantings consist of 4 to 5 seeds per row depending upon species. The smaller seeded species (velvetleaf, wild mustard, pigweed, foxtail millet and green foxtail) are planted in an uncounted but sufficient number to provide a solid row of seedlings.

Plantings for the pre- and post-emergence portions of the test are identical as to seeding. The initial watering until emergence is done from the top. The post-emergence phase is propagated in advance so as to provide plants of the proper stage of development at the time of treatment. Plantings for the pre-emergence phase are made not more than one day in advance of treatment.

The desired stage of development for treatment of the post-emergence broadleaf species (CT, SB, CB, VL, WM, PW) is the one true leaf or first trifoliate leaf stage. The desired stage for corn would be a height of 3—4″, while a 2″ height would be adequate for the grasses.

*Method of Treatment*

Spray applications are made with a handgun sprayer (aspirator type) simultaneously to one flat of established plants for the post-emergence phase and one newly seeded flat for the pre-emergence phase. The 10 lb./acre treatment rate consists of the uniform application of 116 milligrams of test

compound to the combined area of the two flats (160 sq. inches). Application is made in a solvent mixture consisting of 40 ml acetone and 40 ml water and a surfactant concentration of 0.1%.

Following spray application, flats are returned to the greenhouse where watering of the post-emergence phase is done only by subirrigation. The pre-emergence phase is top watered by sprinkling until after test species have emerged. Subsequent watering is by subirrigation.

Two weeks after treatment, the pre- and post-emergence injury and control is rated on a 0—100% injury and control scale. Special physiological effects are rated as to intensify also at this time.

The herbicidal test data reported for compounds 1—3 was obtained at application rates of 2 lbs. down to 1/4 lb./acre. The following lists the metric equivalents for each rate.

Application Rate

| US — lb./acre | Metric — kg/ha |
|---|---|
| 2.0 | 2.24 |
| 0.5 | 0.56 |
| 0.25 | 0.28 |

Test results are set forth in Table I (pre-emergence) and Table II (post-emergence).

TABLE I

| Cpd. No. | Dosage Lbs./ Acre | Pre-Emergence | | | | | | | | | |
|---|---|---|---|---|---|---|---|---|---|---|---|
| | | CG | FM | GF | VL | CB | WM | PW | CT | CN | SB |
| 1 | 2 | 50 | 10 | — | 0 | 10 | 30 | — | — | — | — |
| | 1/4 | 0 | 0 | — | 0 | 0 | 0 | — | — | — | — |
| 2 | 2 | — | 90 | 90 | 70 | 0 | 90 | 80 | 10 | 0 | 0 |
| | 1/2 | — | 70 | 0 | 20 | 0 | 20 | 20 | 0 | 0 | 0 |
| 3 | 2 | — | 80 | 60 | 10 | 10 | 90 | 100 | 20 | 10 | 0 |
| | 1/2 | — | 20 | 20 | 0 | 0 | 10 | 70 | 0 | 0 | 0 |

TABLE II

| Cpd. No. | Dosage Lbs./ Acre | Post-Emergence | | | | | | | | | |
|---|---|---|---|---|---|---|---|---|---|---|---|
| | | CG | FM | GF | VL | CB | WM | PW | CT | CN | SB |
| 1 | 2 | 60 | 50 | — | 90 | 40 | 80 | 100 | 90 | 10 | 30 |
| | 1/4 | 10 | 70 | — | 10 | 10 | 40 | 100 | 40 | 10 | 10 |
| 2 | 2 | 20 | 50 | — | 100 | 70 | 90 | 100 | 80 | 10 | 50 |
| | 1/2 | 10 | 10 | — | 100 | 10 | 90 | 80 | 30 | 10 | 30 |
| 3 | 2 | — | 90 | 90 | 70 | 10 | 100 | 100 | 70 | 50 | 50 |
| | 1/2 | — | 60 | 60 | 10 | 10 | 90 | 90 | 50 | 10 | 40 |
| | 1/4 | — | 60 | 40 | 50 | 20 | 90 | 70 | 30 | 20 | 20 |

**Claims**

1. Herbicidal compounds of the formula wherein

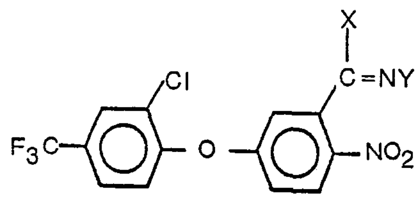

wherein

X is selected from the group consisting of hydrogen, cyano, alkoxy of 1 to 6 carbons, alkyl of 1 to 6 carbons;

Y is selected from the group consisting of hydroxy, alkali metal oxide (e.g., —ONa, —OK), N-alkylaminocarboxy and N,N-dialkylaminocarboxy where each alkyl group is of 1 to 6 carbons, 1-(alkoxycarbonyl) alkoxy where each alkoxy is of 1 to 6 carbons, alkyl of 1 to 6 carbons, epoxyalkoxy where the alkoxy is of 3 to 6 carbons.

2. The compounds of Claim 1 wherein Y is hydroxy.

3. The compounds of Claim 1 wherein Y is an alkali metal oxide.

4. The compounds of Claim 1 wherein Y is selected from N-alkylamino carboxy and N,N-dialkylamino carboxy where each alkyl group is of 1 to 6 carbon atoms.

5. The compound of Claim 4 wherein X is hydrogen.

6. The compounds of Claim 4 wherein each alkyl group is of 1 to 2 carbons and X is hydrogen.

7. The compounds of Claim 1 wherein Y is alkyl of 1 to 6 carbon atoms.

8. The compounds of Claim 1 wherein X is alkyl of 1 to 6 carbon atoms.

9. A herbicidal composition comprising a compound of Claim 1 and a carrier therefor.

10. A method for combating undesirable herbs which comprises contacting them with a herbicidally effective amount of a compound defined in Claim 1.

11. A method for preparing a compound as defined in Claim 1, which comprises reacting an alkanone of the formula

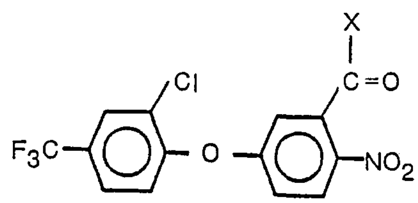

where X is defined in Claim 1 with a compound of the formula $H_2N—Y$ where Y is as defined in Claim 1.

**Revendications**

1. Composés herbicides de formule

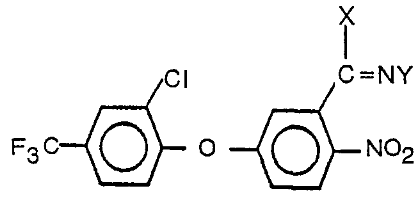

dans laquelle:

X est choisi dans le groupe constitué par l'hydrogène, un groupe cyano, alkoxy de 1 à 6 carbones, alkyle de 1 à 6 carbones

Y est choisi dans le groupe constitué par le groupe hydroxyle, oxyde de métal alcalin tel que —ONa, —OK, N-alkylaminocarboxy et N,N-dialkylaminocarboxy dans lesquels chaque groupe alkyle a de 1 à 6 carbones, (alkoxycarbonyl)-1 alkoxy dans lequel chaque alkoxy a de 1 à 6 atomes de carbone, alkyle de

1 à 6 atomes de carbone, époxyalkoxy dans lequel l'alkoxy a 3 à 6 atomes de carbone.

2. Composés selon la revendication 1 dans laquelle Y est hydroxy.

3. Composés selon la revendication 1 dans laquelle Y est oxyde de métal alcalin.

4. Composés selon la revendication 1 dans laquelle Y est choisi parmi N-alkylaminocarboxy et N,N-dialkylaminocarboxy dans lequel chaque alkyle à 1 à 6 carbone.

5. Composé selon la revendication 4 dans laquelle X est l'hydrogène.

6. Composés selon la revendication 4 dans laquelle chaque alkyl a 1 ou 2 carbones et X est l'hydrogène.

7. Composés selon la revendication 1 dans laquelle Y est alkyle de 1 à 6 atomes de carbone.

8. Composés selon la revendication 1 dans laquelle X est alkyle de 1 à 6 atomes de carbone.

9. Composition herbicide comprenant un composé selon la revendication 1 et un support.

10. Procédé pour combattre les mauvaises herbes qui consiste à les mettre en contact avec une quantité herbicide efficace de composé défini dans la revendication 1.

11. Méthode de préparation de composé tel que défini dans la revendication 1 qui comprend la réaction d'une alcanone de formule

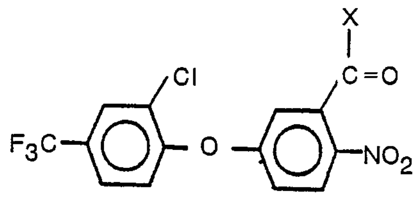

dans laquelle X est tel que défini dans la revendication 1 avec un composé de formule $H_2N—Y$ dans laquelle Y est tel que défini dans la revendication 1.

**Patentansprüche**

1. Herbizide Verbindungen der allgemeinen Formel

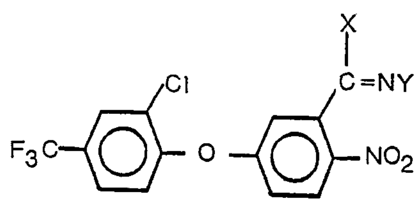

worin X aus einer Gruppe ausgewählt wird, die aus Wasserstoff, Cyan, Alkoxy mit 1 bis 6 Kohlenstoffatomen, Alkyl mit 1 bis 6 Kohlenstoffatomen besteht; Y aus einer Gruppe ausgewählt wird, die aus Hydroxy, Alkalimetalloxid (d.i. —ONa, —OK) N-Alkylaminocarboxy und N,N-Dialkylaminocarboxy, worin jede Alkylgruppe 1 bis 6 Kohlenstoffatome aufweist, 1-(Alkoxycarbonyl)alkoxy, worin jeder Alkoxyrest 1 bis 6 Kohlenstoffatome aufweist, Alkyl mit 1 bis 6 Kohlenstoffatomen, Epoxyalkoxy, worin der Alkoxyrest 3 bis 6 Kohlenstoffatome aufweist, besteht.

2. Verbindungen nach Punkt 1, gekennzeichnet dadurch, daß Y Hydroxy bedeutet.

3. Verbindungen nach Punkt 1, gekennzeichnet dadurch, daß Y ein Alkalimetalloxid darstellt.

4. Verbindungen nach Punkt 1, gekennzeichnet dadurch, daß Y N-Alkylaminocarboxy oder N,N-Dialkylaminocarboxy ist, worin jede Alkylgruppe 1 bis 6 Kohlenstoffatome aufweist.

5. Verbindungen nach Punkt 4, gekennzeichnet dadurch, daß X Wasserstoff darstellt.

6. Verbindung nach Punkt 4, gekennzeichnet dadurch, daß jede Alkylgruppe 1 bis 2 Kohlenstoffatome aufweist und X Wasserstoff ist.

7. Verbindungen nach Punkt 1, gekennzeichnet dadurch, daß Y eine Alkylgruppe mit 1 bis 6 Kohlenstoffatomen darstellt.

8. Verbindungen nach Punkt 1, gekennzeichnet dadurch, daß X eine Alkylgruppe mit 1 bis 6 Kohlenstoffatomen darstellt.

9. Herbizide Zusammenstzung, gekennzeichnet dadurch, daß sie eine Verbindung nach Punkt 1 und einen geeigneten Träger enthält.

10. Verfahren zur Bekämpfung unerwünschten Pflanzenwuchses, gekennzeichnet dadurch, daß man die Pflanzen mit einer herbizid wirksamen Menge einer Verbindung nach Punkt 1 in Kontakt bringt.

11. Verfahren zur Herstellung einer Verbindung nach Punkt 1, gekennzeichnet dadurch, daß man ein Alkanon der allgemeinen Formel

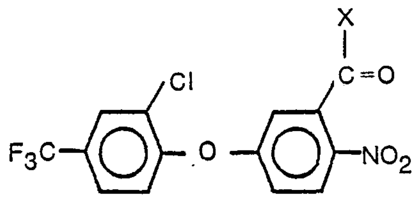

worin X die in Punkt 1 genannte Bedeutung hat, mit einer Verbindung der Formel $H_2N—Y$ umsetzt, wobei Y die in Punkt 1 genannte Bedeutung hat.